# FASCICULE DE BREVET EUROPEEN

(11) **EP 2 733 240 B1**
(45) Date de publication et mention de la délivrance du brevet: **04.03.2015**
(21) Numéro de dépôt: 13189873.6
(22) Date de dépôt: 23.10.2013
(51) Int. Cl.: A61F 13/514, D04H 3/007, D01F 6/46, B32B 5/26, D04H 3/16, D01D 5/098, A61F 13/56, A61F 13/62, A61F 13/15, A61L 15/42, B29C 47/00, A61F 13/496, A61F 13/515, A61L 15/24

(54) **Non-tissé de filaments thermoplastiques filés-liés ayant des propriétés de soudabilité améliorées et procédé de fabrication d'un tel non-tissé**
Spinnvliesstoff aus thermoplastischen Filamenten, die verbesserte Schweißbarkeitseigenschaften haben, und Herstellungsverfahren eines solchen Spinnvliesstoffes
Non-woven fabric of extruded-linked thermoplastic filaments having improved weldability properties and method for manufacturing such a non-woven fabric

(30) Priorité: 15.11.2012 FR 1260877
(43) Date de publication de la demande: 21.05.2014
(73) Titulaire: Dounor, 59535 Neuville en Ferrain (FR)
(72) Inventeur: Hoyas, Stéphanie, 59200 TOURCOING (FR)
(74) Mandataire: Balesta, Pierre

(56) Documents cités:
- EP-A1- 2 028 296
- EP-A2- 0 552 013
- WO-A1-00/20208
- WO-A1-2005/044560
- WO-A1-2005/073309
- WO-A2-2007/140163
- WO-A2-2009/103748
- US-A1- 2010 228 214

## Description

### Arrière-plan de l'invention

La présente invention concerne le domaine technique des non-tissés de filaments thermoplastiques filés-liés ayant des propriétés de soudabilité améliorées ainsi que leur procédé de fabrication.

Dans le domaine de l'hygiène, en particulier concernant les couches-culottes, les garnitures de protection ou autres moyens équivalents, les non-tissés sont généralement fournis aux transformateurs, qui les assemblent selon des zones de solidarisation avec des matériaux complémentaires, par exemple, des matières super-absorbantes ou encore des éléments d'attache, pour former lesdits articles. Les zones de solidarisation, en particulier s'agissant des articles d'hygiène, sont soumises à de nombreux efforts lorsque lesdits articles sont portés. Il est donc indispensable que ces zones de solidarisation résistent lors de l'usage des articles d'hygiène jetables.

Dans un souci d'optimiser le coût de fabrication de tels articles d'hygiène, outre les cadences de solidarisation optimisées, des non-tissés de plus en plus légers permettant l'emploi de quantités de polymères diminuées tout en offrant des propriétés mécaniques satisfaisantes (résistance à la déchirure, résistance à l'abrasion, résistance au peluchage, soudabilité) sont particulièrement recherchés. La diminution de la masse surfacique des non-tissés permet d'obtenir un voile moins cher en réduisant les coûts de transport, de stockage, de production mais également en diminuant la quantité de déchets, et ainsi au final d'obtenir des couches-culottes moins onéreuses. A titre d'exemple, la masse moyenne d'une couche-culotte jetable pour bébé est passée de 65 g au début des années 1980 à moins de 42 g aujourd'hui (tout type de couche-culotte et tout type de tailles confondus), soit une diminution de 30%.

Cependant, plus les non-tissés ont une masse surfacique faible, moins les fibres et/ou filaments qui les composent sont répartis de façon homogène dans leur structure, ce qui engendre des problèmes de reproductibilité, de fiabilité dans la résistance des zones solidarisées, et la présence de zones de faiblesse mécanique.

Les non-tissés peuvent être assemblés selon des zones de solidarisation par une soudure thermique, par ultrasons ou encore par haute fréquence.

Une autre technique de solidarisation des non-tissés entre-eux, consiste à assembler deux voiles non-tissés au moyen d'une fine couche de colle, notamment une colle de type hotmelt. Cette technique a cependant pour inconvénient que la couche de colle doit rester confinée entre les deux non-tissés superposés à assembler et ne doit pas traverser lesdits non-tissés au risque de modifier le toucher et l'aspect des faces externes desdits non-tissés. Lorsque le non-tissé est la couche arrière externe d'une couche culotte, également désigné dans l'état de la technique sous le terme de « backsheet », il est superposé avec un film imperméable aux liquides, lequel film est le plus souvent imprimé. Le film imperméable aux liquides forme alors une couche arrière interne. Ce non-tissé ou couche arrière externe a pour fonction d'éviter que l'utilisateur ne touche le film plastique imperméable et d'attribuer une impression de confort et de douceur à la couche-culotte. Dans ce dernier cas, si la colle passe au travers du non-tissé recouvrant le film, celle-ci risque de détériorer la couche-culotte. Il existe un risque que les couches-culottes collent entre-elles, notamment lors de l'emballage. Ceci pourrait nuire au fonctionnement de la ligne de production, ainsi qu'altérer la fonction première de la couche arrière externe également connue sous le terme de backsheet. On observe que la colle a davantage tendance à transpercer des non-tissés ayant une perméabilité élevée, en particulier s'agissant des non-tissés ayant une faible masse surfacique, i.e au plus de 35 g/m².

En effet, plus les non-tissés ont une masse surfacique faible, plus leur perméabilité augmente. Ces derniers sont ainsi davantage susceptibles d'être transpercés par une colle hotmelt.

On connait le document EP 2.028.296 B1 divulguant un procédé de fabrication d'un non-tissé de filaments filés-liés, également désigné sous le terme de spunbond, ayant pour objet la mise en oeuvre d'une composition comprenant deux polypropylènes ayant des indices d'écoulement à l'état fondu différents, permettant la fabrication de non-tissés ayant des filaments très fins, en particulier inférieurs à 1 dtex. Les non-tissés obtenus ont pour inconvénients qu'ils présentent des propriétés de résistance à la soudure moyenne. On observe en effet que les zones de solidarisation thermo-liées des articles comprenant au moins un desdits non-tissés, par exemples des articles d'hygiène telles que des couches culottes, ont tendance à se dé-laminer après l'opération de soudure, en particulier lorsque la soudure est effectuée à l'aide d'un outil du type thermo-scelleuse.

### Objet et résumé de l'invention

La présente invention a ainsi pour objet, selon un premier aspect, un non-tissé ayant des propriétés de soudabilité améliorées, en particulier ayant une meilleure homogénéité dans la répartition des filaments formant sa structure tout en ayant une résistance mécanique améliorée et une masse surfacique faible.

Ledit non-tissé de filaments thermoplastiques filés-liés comprend de manière caractéristique au moins 95% en masse (g) de sa masse surfacique (g/m²) d'au moins deux polymère(s) de polypropylène A et B, les dits filaments ayant un titrage inférieur ou égal à 1,3 dtex, ledit non-tissé ayant une masse surfacique inférieure ou égale à 35 g/m², le taux de soudure étant au moins de 10% et au plus de 25%. En outre, ledit non-tissé comprend au moins 88% en masse (g) de sa masse surfacique (g/m²) dudit polymère de polypropylène A obtenu par polymérisation en présence d'au moins un catalyseur de polymérisation à base de métallocène et au plus 12% en masse (g) de sa masse surfacique dudit polymère de polypropylène B.

De façon surprenante, le non-tissé selon l'invention présente des propriétés mécaniques et à la soudure thermique améliorées. On entend par soudure thermique, toute soudure réalisée par apport de chaleur, et notamment une soudure ultra-sons, hautes fréquences ou encore à l'aide d'un outil chauffé à l'aide d'une ou plusieurs résistances électriques de préférence à des températures comprises entre 80°C et 160°C. Le non-tissé selon l'invention peut ainsi avantageusement être assemblé en utilisant des machines de solidarisation mettant en oeuvre des mords supérieur et inférieur chauffés à l'aide de résistances électriques et permet ainsi d'améliorer les cadences d'assemblage des articles stratifiés comprenant plusieurs non-tissés sans nuire aux propriétés mécaniques desdits non-tissés.

Le terme filé-lié désigne un non-tissé appelé dans l'état de la technique spunbond et qui est formé par extrusion d'une matière plastique fondue sous forme de filaments à travers les orifices d'une filière, lesdits filaments sont étirés, déposés sur un tapis de nappage, puis liés entre-eux, de préférence par calandrage.

Le terme filé-fondu ou spunmelt désigne tout non-tissé comprenant au moins deux non-tissés de filaments thermoplastiques filés-liés (également désigné dans le présent texte sous le terme de spunbond) et au moins un non-tissé de fibres fondues-soufflées (également désigné dans le présent texte sous le terme de meltblown).

Les polymères de polypropylène A et B sont des homopolymères de propylène ou des copolymères de propylène et d'autres alpha-oléfines. Des exemples d'alpha-oléfines qui peuvent être co-polymérisés comprennent les alpha-oléfines de 2 à 20 atomes de carbone, tel que l'éthylène, le 1-butène, le 1-pentène, le 1-hexène, le 1-octène, le 1-decène, le 3-méthyl-1-butène, le 3-méthyl-1-pentène, le 3-éthyl-1-pentène, le 4-méthyl-1-pentène et le 4-méthyl-1-hexène. L'éthylène et le 1-butène sont préférés, en particulier l'éthylène. De tels alpha-oléfines peuvent être polymérisés seuls ou en combinaison à deux ou plus.

On comprend par thermoplastique, toute matière plastique, dans le cas de la présente invention des polymères de polypropylène, capables de fondre sous l'action de la chaleur ou, tout au moins, de se ramollir suffisamment pour pouvoir être mis en forme plusieurs fois sans altération significative de ses propriétés mécaniques.

On comprend par non-tissé, toute nappe de fibres et/ou de filaments entrelacés de façon aléatoire, à la différence par exemple d'un tissu ou d'un tricot.

On comprend par sens machine, la direction du non-tissé correspondant à la direction selon laquelle il a été produit, en général par référence à sa disposition sur le tapis convoyeur lors de l'étape de consolidation, en particulier lors du calandrage. Le sens travers correspond à la direction perpendiculaire au sens machine.

Le taux de soudure d'un non-tissé est le ratio de la surface partielle correspondant à la zone dans laquelle les fibres/filaments sont liés, en particulier calandrés, par rapport à la surface totale dudit non-tissé. Généralement, les voiles de filaments thermoplastiques filés sont liés par calandrage, le taux de soudure dépend donc des motifs de gravure en surface du cylindre destiné au calandrage.

Ce taux de soudure peut ainsi être calculé soit à partir des gravures figurant sur le cylindre gravé mis en oeuvre lors du calandrage ou à partir du non-tissé fini. Dans ce dernier cas, un scanner à plat est utilisé pour scanner en haute résolution l'une des deux surfaces dudit non-tissé. Une méthode de mesure précise est décrite dans le document US 2012/0179125 aux paragraphes [0222] et suivants.

Les catalyseurs de polymérisation Ziegler-Natta des homopolymères ou copolymères de propylène sont bien connus de l'homme du métier. Les mécanismes de polymérisation sont par exemple décrits dans « Encyclopedia of Polymer Science and Engineering », volume 8, page 162, publié par John Wiley and Sons, Inc, 1987.

Les polymères de polypropylène décrits ci-après comme étant obtenus par polymérisation en présence d'un catalyseur métallocène sont produits selon un procédé dit métallocène. De tels polymères de polypropylène sont commercialisés par Exxon Chemical Company sous la marque commerciale Exxon Achieve TM, par Total Petrochemicals sous la marque LUMICENE®, ou encore par Lyondell Basell sous la marque METOCENE®.

Le polymère de polypropylène B peut être obtenu par polymérisation en présence d'un catalyseur de polymérisation à base de métallocène et/ou Ziegler-Natta.

Les polymères de polypropylène A et B sont différents, notamment en ce qu'ils ont des indices à l'état fondu différents, respectivement MFI (Melt Flow Index) (A) et MFI (Melt Flow Index) (B), mesurés d'après la norme ISO 1133, condition L, à 230°C et 2,16 Kg, de préférence MFI (B) est supérieure à MFI (A), encore de préférence le MFI (B) est supérieure d'au moins 15 fois au MFI (A).

De préférence, le polymère de polypropylène A a un indice d'écoulement à l'état fondu d'au moins 10 g/10 min et au plus de 35 g/10 min, et le polymère de polypropylène B a un indice d'écoulement à l'état fondu d'au moins 600 g/10 min et au plus de 3 000 g/10 min , l'indice d'écoulement à l'état fondu étant déterminé d'après la norme ISO 1133, condition L, à 230°C et 2,16 Kg.

De préférence, le non-tissé selon l'invention comprend au plus 7% en masse par rapport à sa masse surfacique du polymère de polypropylène B, encore de préférence entre 3% et 6% en masse de sa masse surfacique du polymère de polypropylène B.

Dans une variante, ledit non-tissé comprend au moins 93% en masse de sa masse surfacique dudit polymère de polypropylène A.

Les inventeurs se sont aperçus que l'augmentation de la quantité de polypropylène A dans la composition extrudable de départ lors de la fabrication du non-tissé selon l'invention, et donc se trouvant au final au moins dans une proportion en masse par rapport à sa masse surfacique supérieure ou égale à 93%, permet d'améliorer les performances mécaniques, en particulier ses résistances à la rupture dans les sens travers et machine.

En outre, cette proportion en polymère de polypropylène A combinée avec le polymère de polypropylène B permet d'améliorer considérablement les propriétés de résistance à la soudure du non-tissé selon l'invention.

Dans une variante, les filaments ont un titrage compris entre 0,01 dtex et 1,2 dtex, de préférence entre 0,5 dtex et 1,1 dtex, encore de préférence entre 0,5 dtex et 1 dtex, et encore de préférence entre 0,7 dtex et 1 dtex.

De préférence, lorsque le non-tissé est utilisé dans la fabrication d'un article d'hygiène, en particulier pour les couches-culottes, et notamment en tant que couche arrière externe ou « backsheet », les filaments ont un titrage inférieur ou égal à 1,2 dtex, de préférence inférieur ou égal à 1,1 dtex, encore de préférence inférieure ou égal à 1 dtex.

Avantageusement, la composition de polymères de polypropylène A et B selon l'invention permet d'obtenir des filaments très fins, et donc une longueur de filaments plus importante au m² que dans un non-tissé de même masse surfacique mais ayant des filaments de titrage supérieur à 1,3 dtex.

Dans une variante, le non-tissé selon l'invention a une masse surfacique d'au moins 5g/m² et d'au plus 30 g/m², de préférence d'au moins 10 g/m² et d'au plus 25 g/m².

Dans une variante, ledit non-tissé comprend au moins 9,5 Km de filaments par m².

Cet intervalle de longueur de filaments est calculé d'après le titrage desdits filaments et la masse surfacique du non-tissé. Cet intervalle de longueur de filaments filés-liés par gramme de non-tissé et par m² permet d'obtenir des résistances dans le sens machine et le sens travers pour le non-tissé nettement améliorées comparativement à un non-tissé du type spunbond composé d'un seul polymère de polypropylène obtenu par polymérisation en présence d'un catalyseur de polymérisation Ziegler-Natta.Dans une variante, ledit non-tissé a une perméabilité (l/m²/s) mesurée selon la norme ISO 9237 datant de 1995 inférieure à 6500 l/m²/s, de préférence inférieure à 6100 l/m²/s.

Avantageusement, le non-tissé selon l'invention conserve une perméabilité restreinte. Une explication non exhaustive serait que les filaments étant très fins, le nombre de km de filaments par m² augmente comparativement à des filaments de plus gros titrage, assurant ainsi une bonne répartition des filaments dans la structure du non-tissé.

La présente invention a pour objet, selon un deuxième aspect, un article comprenant au moins un premier non-tissé selon l'une quelconque des variantes de réalisation précédentes, et au moins un second non-tissé comprenant des fibres et/ou des filaments à base d'un polymère de polypropylène, lesdits premier et second non-tissés sont solidarisés selon au moins une zone de solidarisation dans laquelle ils sont thermo-liés.

Ledit second non-tissé peut être un non-tissé de type spunbond, un spunmelt (c'est-à-dire comprenant au moins deux spunbond et un meltblown), un meltblown, un voile cardé, thermo-soudé, aiguilleté, spunlacé ou encore une combinaison de ces derniers.

De préférence, le second non-tissé est un premier non-tissé selon l'invention.

Ledit article est de préférence jetable, et peut être un article d'hygiène tel qu'une couche-culotte, un dispositif cataménial, un dispositif pour l'incontinence adulte ou encore des masques respiratoires, des blouses chirurgicales ou vêtements de protection, des casaques,... nécessitant l'emploi d'au moins une zone de solidarisation dans laquelle les fibres et/ou filaments sont thermo-liés.

Ladite zone de solidarisation peut être obtenue à l'aide d'une soudure thermique entraînant la fusion au moins partielle des fibres/filaments en polypropylène : thermo-soudure à l'aide d'une thermo-scelleuse, soudure ultra-sons, soudure à hautes fréquences. On comprend par « thermo-liés », le fait de lier un non-tissé sur lui-même ou avec un autre non-tissé par apport de chaleur suffisant pour entraîner la fusion totale ou partielle des fibres/filaments dans la zone de solidarisation.

Ladite zone de solidarisation peut correspondre à une zone limitée des deux faces solidarisées et en contact des premier et second non-tissés ou à toute la surface desdites faces en contact.

De préférence, la soudure est une soudure thermique et la zone de solidarisation comprend des fibres/filaments au moins partiellement fondus.

Dans une variante, ladite zone de solidarisation de l'article comprend au moins deux premiers non-tissés, de préférence au moins quatre premiers non-tissés.

La présente invention a pour objet, selon un troisième aspect, un article d'hygiène, tel qu'une couche-culotte, adapté pour être porté autour de la taille d'un porteur, ayant un axe longitudinal (L), et comprenant :
- une couche avant perméable aux liquides,
- une couche arrière imperméable aux liquides, et
- une couche absorbante disposée entre les couches avant et arrière.

Avantageusement la couche arrière imperméable aux liquides comprend un film polymère imperméable aux liquides orienté vers ledit porteur et un premier non-tissé selon l'une quelconque des variantes de réalisation précédentes.

La couche absorbante a pour fonction d'absorber et de stocker les liquides. Cette couche absorbante est composée de fluff et de polymères super-absorbants, lesquels sont des polymères réticulés qui peuvent absorber au moins cinquante fois leur poids d'une solution saline à 0,9% selon le test de mesure de la capacité de rétention (EDANA 441.2-01). Le film polymère imperméable aux liquides est de préférence extrudé-calandré et comprend un ou plusieurs des polymères suivants de préférence fusible(s) : polypropylène, polyéthylène, polyamide, polyéthylènetérephtalate, polyuréthane.

Dans une variante, le film polymère imperméable aux liquides et ledit premier non-tissé sont assemblés selon au moins une zone de solidarisation dans laquelle ledit film et ledit premier non-tissé sont thermo-liés.

Ladite zone de solidarisation est définie telle que décrit ci-dessus.

Dans une variante, ledit article d'hygiène comprend un élément d'attache solidarisé avec ledit premier non-tissé selon une zone de solidarisation dans laquelle ledit élément d'attache et ledit premier non-tissé sont thermo-liés.

Ladite zone de solidarisation est définie telle que décrit ci-dessus.

Les éléments d'attache décrits dans la présente invention comprennent selon une portion de leurs faces internes des organes d'attache, par exemple des crochets (par exemple en forme de T, de champignons,...etc) aptes à coopérer pour leur attache avec des boucles d'attache formées par les fibres et/ou filaments se projetant de la face externe de la couche arrière de l'article d'hygiène.

Dans une variante, la couche arrière de l'article d'hygiène comprend des portions avant et arrière solidarisées selon des zones de solidarisation latérales dans lesquelles lesdites portions avant et arrière sont thermo-liées.

Lesdites zones de solidarisation latérales sont définies telle que décrit ci-dessus en référence à au moins une zone de solidarisation.

Dans une variante, la couche arrière comprend au moins deux premiers non-tissés selon l'une quelconque des variantes de réalisation précédentes.

L'invention a pour objet, selon un quatrième aspect, un procédé de fabrication d'un non-tissé de filaments thermoplastiques filés-liés selon l'une des variantes de réalisation précédentes, comprenant les étapes suivantes :
a. Extruder à travers les orifices d'une filière une composition à base de polypropylène comprenant au moins 95% en poids de son poids d'un mélange de deux polymères de polypropylène A et B, le polymère de polypropylène A étant présent à une teneur d'au moins 88 % en poids par rapport au poids de ladite composition et ayant un indice d'écoulement à l'état fondu d'au moins 10 g/10 min et au plus de 35 g/10 min, et le polymère de polypropylène B étant présent à une teneur d'au plus 12% en poids par rapport au poids de ladite composition et ayant un indice d'écoulement à l'état fondu d'au moins 600 g/10 min et au plus de 3 000 g/10 min , l'indice d'écoulement à l'état fondu étant déterminé d'après la norme ISO 1133, condition L, à 230°C et 2,16 Kg,
b. Etirer et napper les filaments extrudés sur un support,
c. Consolider lesdits filaments, notamment par calandrage, pour la formation d'un non-tissé.

De manière caractéristique, le polypropylène A est obtenu par polymérisation en présence d'un catalyseur de polymérisation à base de métallocène.

La demanderesse s'est aperçue que cette composition particulière permet d'obtenir un non-tissé ayant un comportement amélioré à la soudure ou d'améliorer le comportement à la soudure d'un article stratifié comprenant au moins un non-tissé selon l'invention et un ou plusieurs non-tissés différents, par exemple du type spunmelt et ce pour des non-tissés de faible masse surfacique, notamment inférieure ou égale à 35 g/m². Par comportement amélioré à la soudure, on comprend l'amélioration de la résistance à la délamination ou à la déchirure d'une zone de solidarisation comprenant au moins un non-tissé selon l'invention.

La composition selon l'invention peut comprendre des additifs usuels, tels que des antioxydants, des anti-acides, des anti-UV, des colorants, des matières de charge, des agents antistatiques, des agents lubrifiants et des agents favorisant le glissement. Ces additifs sont généralement ajoutés par mélange à l'état fondu, par exemple lors de fabrication des granulés à partir de la composition selon l'invention. Ces granulés sont ensuite fondus en vue de l'extrusion-filage sur une filière d'extrusion pour la fabrication d'un voile non-tissé spunbond. Le voile est ensuite consolidé de préférence par calandrage. Cette opération consiste à faire passer le voile non encore consolidé sur un jeu de cylindres gravés, de préférence chauffés, en sorte de créer des zones de solidarisation inter-filamentaires et des zones non solidarisées pour laisser du gonflant et de la douceur audit non-tissé.

La teneur totale en additifs ne dépasse généralement pas 5% en poids du poids total de la composition selon l'invention, de préférence elle est inférieure à 2% en poids, et encore de préférence inférieure à 1% en poids.

De préférence, la composition selon l'invention comprend au moins 95% en poids de son poids du mélange des polymères de polypropylène A et B, de préférence au moins 98% en poids, et encore de préférence au moins 99% en poids.

L'indice à l'état fondu indiqué dans le cadre du présent texte est celui mesuré sur le polymère de polypropylène entrant dans la composition selon l'invention avant son extrusion-filage pour la fabrication du non-tissé.

Dans une variante, la composition comprend au moins 93% en poids dudit polymère de polypropylène A, et au plus 7% en poids dudit polymère de polypropylène B, de préférence de l'ordre de 3% à 6% en poids dudit polymère de polypropylène B.

Dans une variante, le polymère de polypropylène B est obtenu par polymérisation en présence d'un catalyseur de polymérisation à base de métallocène et/ou Ziegler-Natta, de préférence Ziegler-Natta.

Dans une variante, le procédé de fabrication comprend les étapes suivantes pour la fabrication d'un article selon l'une quelconque des variantes de réalisation précédentes :
d. Superposer au moins un premier non-tissé obtenu à l'issue de l'étape c), et un second non-tissé,
e. Solidariser à l'aide d'un outil chauffé à une température comprise entre 150°C et 158°C, de préférence entre 153°C et 155°C, lesdits au moins deux non-tissés selon une zone de solidarisation dans laquelle les dits non-tissés sont thermo-liés.

La demanderesse a déterminé que cet intervalle de température permet d'améliorer la résistance à la délamination de la zone de solidarisation.

Le second non-tissé et la zone de solidarisation sont définis ci-dessus.

### Brève description des dessins

La présente invention sera mieux comprise à la lecture des exemples de réalisation, cités à titre non limitatif, et des figures suivantes dans lesquelles :
- La figure **1** représente de façon schématique et en perspective une première variante d'un article d'hygiène, en particulier une couche-culotte pull up dont la couche arrière comprend au moins un non-tissé selon l'invention ;
- La figure **2** représente de façon schématique et en perspective une seconde variante d'un article d'hygiène, en particulier une couche-culotte dans laquelle la couche arrière comprend au moins deux non-tissés selon l'invention ;
- La figure **3** représente de façon schématique et vue de dessus à plat l'article d'hygiène représenté à la figure **2****.**

### Description détaillée de l'invention

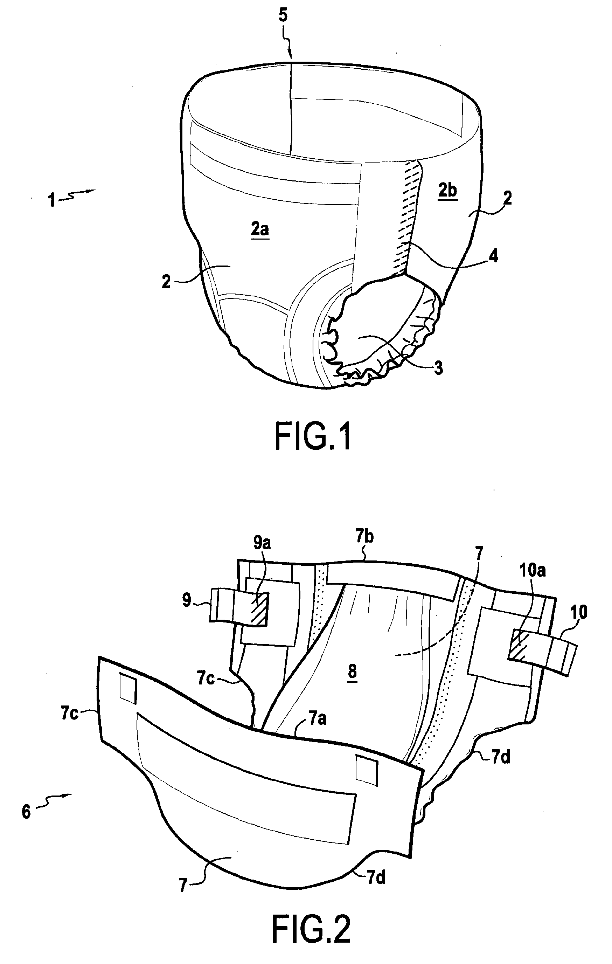

La couche-culotte **1** représentée à la figure **1** est jetable et destinée aux bébés. Cette couche-culotte **1** comprend une couche arrière **2** et une couche avant **3** entre-lesquelles est disposée une couche absorbante. La couche arrière **2** comprend au moins deux premiers non-tissés selon l'invention. Les portions avant **2a** et arrière **2b** de la couche arrière **2** sont solidarisées selon des zones de solidarisation latérales **4** et **5** dans lesquelles les filaments en polymères de polypropylène sont au moins partiellement fondus. Cette couche-culotte **1** est également dans l'état de la technique désignée sous le terme de « training pant » et est disposée sur la taille de l'utilisateur en passant ses jambes à travers les deux ouvertures.

La couche-culotte **6** représentée à la figure **2** est également jetable et destinée aux bébés. Cette couche-culotte comprend une couche avant **8** et une couche arrière **7** entre lesquelles est disposée une couche absorbante non représentée. La couche arrière **7** comprend au moins un non-tissé selon l'invention, de préférence au moins deux non-tissés selon l'invention. Les bordures avant **7a,** arrière **7b** et latérales **7c** et **7d** sont de préférence assemblées par apport de chaleur, en particulier à l'aide d'une thermo-scelleuse ou par soudure ultra-sons ou hautes fréquences. La couche-culotte **6** comprend également deux éléments d'attache **9** et **10** solidarisés à la couche arrière **7** selon les zones de solidarisation **9a** et **10a** par apport de chaleur, et notamment à l'aide d'une thermo-scelleuse, d'ultra-sons ou de hautes-fréquences.

La couche arrière **7** comprend un film imperméable aux liquides et perméable à l'air en tant que couche arrière interne et au moins un non-tissé selon l'invention, de préférence au moins deux non-tissés selon l'invention, recouvrant ledit film et formant une couche arrière externe. La couche arrière interne formée du film imperméable aux liquides est orientée au regard du porteur de la couche culotte **1** tandis que la couche arrière externe formée d'au moins un non-tissé selon l'invention vient en contact avec la personne qui dispose la couche-culotte sur le porteur.

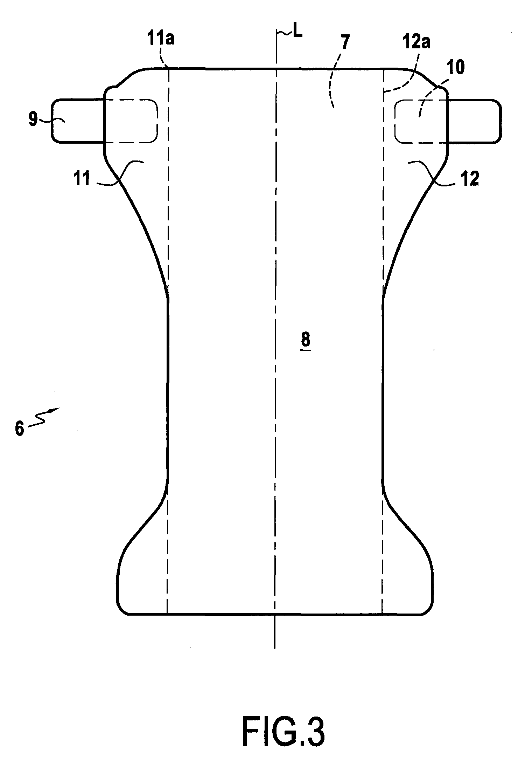

Dans la variante de la couche-culotte **6** représentée à la figure **2****,** les éléments d'attache **9** et **10** sont solidarisés directement à la couche arrière **7** mais ces derniers pourraient être solidarisés à des pattes d'attache intermédiaires **11** et **12** délimitées par rapport à la couche arrière **7** par des pointillés représentés sur la figure **3****.** Dans ce dernier cas, les pattes d'attache intermédiaires **11** et **12** sont solidarisées selon les zones de solidarisation **11a** et **12a** par apport de chaleur sur la couche arrière **7.** Les éléments d'attache **9** et **10** sont quant à eux solidarisés de la même façon aux pattes intermédiaires d'attache **11** et **12** qu'à la couche arrière **7.** De préférence, les pattes d'attache intermédiaires **11** et **12** comprennent au moins un non-tissé selon l'invention, de préférence aux moins deux non-tissés selon l'invention.

Les tableaux 1 à 4 ci-après reprennent les résultats d'essais effectués sur différents non-tissés filés-liés (spunbond) selon l'invention et de l'état de la technique, et les articles les combinant.

**Tableau 1**

| Paramètres de soudage | Exemple 1 / Force à rupture | Exemple 2 / Force à rupture | Exemple 3 / Force à rupture | Exemple 4 / Force à rupture | Exemple 5 / Force à rupture | Exemple 6 / Force à rupture | Exemple 7 / Force à rupture | Exemple 8 / Force à rupture |
|---|---|---|---|---|---|---|---|---|
| T°C : 150°C | 8.6 N/5cm | 8.3 N/5cm | 8.8 N/5cm | 4.5 N/5cm | 13 N/5cm | 7.8 N/5cm | 10.5 N/5cm | 3.2 N/5cm |
| Force : 150N (=3 bars) | | | | | | | | |
| Temps : 1 seconde | | | | | | | | |
| T°C : 153°C | 15 N/5cm | 15.1 N/5cm | 17 N/5cm | 6.7 N/5cm | 35 N/5cm | 29 N/5cm | 29.3 N/5cm | 5.4 N/5cm |
| Force : 150N (=3 bars) | | | | | | | | |
| Temps : 1 seconde | | | | | | | | |
| T°C : 155°C | 22.6 N/5cm | 25.5 N/5cm | 27.3 N/5cm | 12.1 N/5cm | 56.1 N/5cm | 50.7 N/5cm | 47.5 N/5cm | 14.8 N/5cm |
| Force : 150N (=3 bars) | | | | | | | | |
| Temps : 1 seconde | | | | | | | | |

L'exemple 1 est un article comprenant un spunbond selon l'invention de 16 g/m² (désigné dans la suite du présent texte comme spunbond mixed polymer) obtenu par la mise en oeuvre de la composition selon l'invention comprenant un mélange de deux polymères de polypropylène A et B, le polymère de polypropylène A étant présent à une teneur d'au moins 93% en poids par rapport au poids de ladite composition, dans cet exemple précis de l'ordre de 95% en poids, et ayant un indice d'écoulement à l'état fondu de 27 g/10 min et le polymère de polypropylène B étant présent à une teneur d'au plus 7% en poids par rapport au poids de ladite composition, dans cet exemple précis d'environ 5% en poids, et ayant un indice d'écoulement à l'état fondu de 1200 g/10 min. Ledit article comprend également, superposés dans cet ordre sur le spunbond mixed polymer de 16g/m², deux spunmelt (Spunbond/ Meltblown / Spunbond) de chacun 15 g/m² et un spunbond mixed polymer de 16 g/m². Les quatre couches de non-tissés ont été individuellement calandrées à l'aide d'un cylindre gravé et chauffé en sorte que le ratio de la surface des zones soudées par rapport à la surface des non-tissés soit inférieur ou égal à 25%, de préférence de l'ordre de 20%. Les dessins de la gravure sont en T pour les spunbond mixed polymer. De préférence, les polymères de polypropylène A et B dans ladite composition sont des homopolymères. La masse surfacique de l'article obtenu est de 62 g/m². Les spunbond mixed polymer ont dans cet exemple précis des filaments ayant un titrage de 1,17 dtex à plus ou moins 5% près en moyenne. Le spunbond du spunmelt comprend des filaments ayant un titrage de 1,7 dtex et des fibres d'environ 2 µm pour le meltblown. Dans le présent texte, les fibres des non-tissés meltblown ont un diamètre d'environ 2 µm et un titrage de 0,03 dtex.

Les exemples 2 à 21 sont décrits de par leurs différences avec l'exemple 1, ils présentent également un taux de soudure de 16 à 22%.

L'exemple 2 est un article de 62 g/m² comprenant dans cet ordre, superposés, un spunbond mixed polymer de 16 g/m² (1,08 dtex / filament), deux spunmelt de 15 g/m² chacun (1,89 dtex / filament / spunbond), et un spunbond mixed polymer de 16 g/m² (1,08 dtex / filament). Les dessins de la gravure sont des pointes de diamant pour les spunbond mixed polymer.

L'exemple 3 est un article de 66 g/m² comprenant dans cet ordre, superposés, un spunbond mixed polymer de 18 g/m² (1,09 dtex / filament), deux spunmelt de 15 g/m² chacun (1,89 dtex / filament / spunbond), et un spunbond mixed polymer de 18 g/m² (1,09 dtex / filament). Les dessins de la gravure sont des pointes de diamant pour les spunbond mixed polymer.

L'exemple 4 est un article de 70 g/m² comprenant dans cet ordre, superposés, un spunbond standard de 20 g/m² (2,11 dtex / filament), deux spunmelt de 15 g/m² chacun (1,89 dtex / filament / spunbond), un spunbond standard de 20 g/m² (2,11 dtex / filament). Les dessins de la gravure sont T pour le spunbond standard.

L'exemple 5 est un article de 64 g/m² comprenant quatre spunbond mixed polymer de 16 g/m² chacun (1,17 dtex / filament). Les dessins de la gravure étant en T.

L'exemple 6 est un article de 64 g/m² comprenant quatre spunbond mixed polymer de 16 g/m² chacun (1,08 dtex / filament). Les dessins de la gravure sont des pointes de diamant.

L'exemple 7 est un article de 72 g/m² comprenant quatre spunbond mixed polymer de 18 g/m² chacun (1.09 dtex / filament). Les dessins de la gravure sont des pointes de diamant.

L'exemple 8 est un article de 80 g/m² comprenant quatre spunbond standards de 20 g/m² chacun (2,11 dtex / filament). Les dessins de soudure sont des T.

La première colonne du tableau 1 indique les paramètres de soudure pour les exemples 1 à 8. Les articles décrits aux exemples 1 à 8 ont ainsi été soudés selon une zone de solidarisation donnée au moyen d'une soudeuse comprenant des mors supérieur et inférieur chauffés. Les températures des mors sont indiquées à la première colonne ainsi que les pressions exercées par les deux mors, et le temps de soudure (lequel est toujours d'une seconde).

Les résistances à rupture en Newtons/5cm indiquées dans les colonnes restantes du tableau 1 résultent de la moyenne de trois essais de traction à rupture effectués de part et d'autre de ladite zone de solidarisation. Les deux parties solidarisées des articles se projetant de part et d'autre de la zone de solidarisation sont disposées entre les mors supérieur et inférieur d'un banc de traction de type Lloyd et distantes de 10 cm. La vitesse de traction est de 300 mm/min. Les quatre non-tissés testés ont une largeur de 50 mm et une longueur de 200 mm. La machine de soudure utilisée est une thermo-scelleuse OTTO BRUGGER HSG/C.

A la lecture des résultats figurant au tableau 1, on observe que la force à rupture de la zone de solidarisation est améliorée dès lors que l'article comprend au moins un non-tissé de filaments filés-liés selon l'invention (spunbond) comparativement aux articles comprenant des spunbond standards, et ce quelle que soit la température à laquelle les quatre couches de non-tissés sont soudés dans ladite zone. Cette force à rupture est améliorée lorsque l'article comprend deux non-tissés spunbond mixed polymer, et encore améliorée lorsqu'il comprend quatre non-tissés spunbond mixed polymer, et toujours quelle que soit la température à laquelle les quatre non-tissés sont assemblés dans ladite zone.

On remarque également que la température à laquelle la zone de solidarisation est formée influence la force à rupture obtenue. Par exemple, la force à rupture (N/5cm) est multipliée par trois pour l'article de l'exemple 6 entre 150°C et 153°C, et multipliée par cinq entre 150°C et 155°C.

**Tableau 2**

| Paramètres de soudage | Exemple 9 / Force à rupture | Exemple 10 / Force à rupture | Exemple 11 / Force à rupture | Exemple 12 / Force à rupture |
|---|---|---|---|---|
| T°C : 150°C | 15,7 N/5cm | 2,7 N/5cm | 7,8 N/5cm | 4,3 N/5cm |
| Force : 150N (=3 bars) | | | | |
| Temps : 1 seconde | | | | |
| T°C : 153°C | 40,4 N/5cm | 7,2 N/5cm | 29 N/5cm | 6,7 N/5cm |
| Force : 150N (=3 bars) | | | | |
| Temps : 1 seconde | | | | |
| T°C : 155°C | 28,9 N/5cm | 20,4 N/5cm | 50,7 N/5cm | 13,8 N/5cm |
| Force : 150N (=3 ba rs) | | | | |
| Temps : 1 seconde | | | | |
| T°C : 158°C | - | 25,6 N/5cm | 60,2 N/5cm | 24,4 N/5cm |
| Force : 150N (=3 bars) | | | | |
| Temps : 1 seconde | | | | |

L'exemple 9 est un article de 60 g/m² comprenant quatre non-tissés spunbond de 15 g/m² (2,44 dtex / filament), consistant en une composition comprenant un seul polymère de polypropylène obtenu par polymérisation en présence d'un catalyseur de polymérisation métallocène, dans cet exemple précis il s'agit du polymère de polypropylène A.

L'exemple 10 est un article de 60 g/m² comprenant quatre non-tissés spunbond de 15 g/m² (2,4 dtex / filament), consistant chacun en une composition comprenant un seul polymère de polypropylène obtenu par polymérisation en présence d'un catalyseur de polymérisation Ziegler-Natta.

L'exemple 11 est un article de 64 g/m² comprenant quatre non-tissés (1,08 dtex / filament) selon l'invention (spunbond mixed polymer), en particulier consistant chacun en une composition comprenant le polymère A et le polymère B.

L'exemple 12 est un article de 60 g/m² comprenant quatre non-tissés spunmelt, en particulier chaque non-tissé est un voile du type SMS (Spunbond/Meltblown/Spunbond) de 15 g/m² (1,89 dtex / filament / spunbond) consistant chacun en une composition comprenant un seul polymère de polypropylène obtenu par polymérisation en présence d'un catalyseur de polymérisation Ziegler-Natta.

Les paramètres de soudure de la zone de solidarisation des articles stratifiés des exemples 9 à 12 sont les mêmes que ceux décrits pour les exemples 1 à 8.

A la lecture du tableau 2, on observe que l'article de l'exemple 9 ne résiste pas à une température de soudure de 158°C puisque l'article est totalement fondu au contact des mors de la thermo-scelleuse. Ainsi, l'utilisation d'un seul polymère de polypropylène A pour la fabrication d'un non-tissé spunbond ne permet pas d'obtenir des forces à rupture élevées et reproductibles, il est nécessaire d'utiliser également un polymère de polypropylène B sélectionné selon l'invention.

Les articles des exemples 10 et 12 résistent à une température de soudure de 158°C mais ne permettent pas d'atteindre des forces à rupture aussi élevées que celles obtenues pour l'exemple 11 comprenant quatre non-tissés selon l'invention.

**Tableau 3**

| Paramètres mesurés | Exemple 13 | Exemple 14 | Exemple 15 |
|---|---|---|---|
| Force à rupture (Newtons) / sens machine WSP 110.4(09) | 37 N/5cm | 25 N/5cm | 27.5 N/5cm |
| Force à rupture (Newtons) / sens travers WSP 110.4(09) | 22.6 N/5cm | 14.6 N/5cm | 17.7 N/5cm |
| Allongement (%) sens machine WSP 110.4(09) | 75 % | 50.1% | 77.7 % |
| Allongement (%) sens travers WSP 110.4(09) | 84.5 % | 52.9 % | 80.7 % |
| Titrage d'un filament (dtex) | 1.05 dtex | 1.15 dtex | 2 dtex |
| Perméabilité à l'air (l/m²/s) norme ISO 9237 | 5100 l/m²/s | 5900 l/m²/s | 7400 l/m²/s |
| Longueur de filaments par m² en kilomètres | 114 | 104 | 60 |

L'exemple 13 correspond à un non-tissé spunbond mixed polymer selon l'invention de 12 g/m² dont la proportion en poids des polymères de polypropylène A et B est respectivement de 95% et de 5% par rapport au poids de la composition extrudable selon l'invention. Les proportions en additifs de l'ordre de 0,5% à 1% en poids par rapport au poids de la dite composition n'ont pas été comptabilisés.

L'exemple 14 correspond à un non-tissé spunbond mixed polymer de 12 g/m² dont la proportion en poids des polymères de polypropylène A et B est respectivement de 90% et de 10% dans la composition selon l'invention. Les proportions en additifs de l'ordre de 0,5% à 1% en poids par rapport au poids de la dite composition n'ont pas été comptabilisés.

L'exemple 15 correspond à un non-tissé standard de 12 g/m² (un seul polymère de polypropylène obtenu par polymérisation en présence d'un catalyseur de polymérisation Ziegler-Natta).

On observe que plus la quantité de polymère de polypropylène A augmente, plus le titrage des filaments diminue. Corrélativement, la longueur des filaments en kilomètre par m² de non-tissé augmente également.

Avantageusement, on observe que les exemples 13 et 14 ont également une perméabilité à l'air plus faible à poids égal comparativement à l'exemple 15. Les forces à rupture dans les sens machine et travers sont nettement améliorées pour l'exemple 13 comparativement aux exemples 14 et 15.

**Tableau 4**

| Paramètres de soudage | Exemple 16 / Force à rupture | Exemple 17 / Force à rupture | Exemple 18 / Force à rupture | Exemple 19 / Force à rupture | Exemple 20 / Force à rupture | Exemple 21 / Force à rupture |
|---|---|---|---|---|---|---|
| T°C : 150°C | 3.1 N/5cm | 9.7 N/5cm | 17.4 N/5cm | 13.9 N/5cm | 11.9 N/5cm | 15.1 N/5cm |
| Force : 150N (=3 bars) | | | | | | |
| Temps : 1 seconde | | | | | | |
| T°C : 153°C | 5.6 N/5cm | 24.5 N/5cm | 28.1 N/5cm | 21.9 N/5cm | 44 N/5cm | 41.4 N/5cm |
| Force : 150N (=3 bars) | | | | | | |
| Temps : 1 seconde | | | | | | |
| T°C : 155°C | -- | -- | -- | -- | 54.9 N/5cm | 28.4 N/5cm |
| Force : 150N (=3 bars) | | | | | | |
| Temps : 1 seconde | | | | | | |
| T°C : 158°C | -- | -- | -- | -- | 35 N/5cm | 26.2 N/5cm |
| Force : 150N (=3 bars) | | | | | | |
| Temps : 1 seconde | | | | | | |

Les exemples 16 à 21 sont décrits de par leurs différences avec l'exemple 1 et testés de la même façon concernant la force à rupture de la zone de solidarisation que pour l'exemple 1. Les proportions en additifs de l'ordre de 0.1 à 0.5% en poids par rapport au poids de la composition extrudable n'ont pas été comptabilisées dans les valeurs données dans les exemples 16 à 21. L'exemple 16 est un article de 40 g/m² comprenant quatre non-tissés spunbond mixed polymer de 10 g/m² (0,97 dtex / filament) selon l'invention, la composition extrudable comprend 90% en poids de son poids d'un polymère de polypropylène A obtenu par polymérisation en présence d'un catalyseur de polymérisation Ziegler-Natta et 10% en poids de son poids d'un polymère de polypropylène B obtenu par polymérisation en présence d'un catalyseur de polymérisation Ziegler-Natta.

L'exemple 17 est un article de 40 g/m² comprenant quatre non-tissés spunbond mixed polymer de 10 g/m² (0,81 dtex / filament), la composition extrudable comprend 90% en poids de son poids d'un polymère de polypropylène A obtenu par polymérisation en présence d'un catalyseur de polymérisation à base de métallocène et 10% en poids de son poids d'un polymère de polypropylène B obtenu par polymérisation en présence d'un catalyseur de polymérisation Ziegler-Natta.

L'exemple 18 est un article de 40 g/m² comprenant quatre non-tissés spunbond mixed polymer de 10 g/m² (1,02 dtex / filament), la composition extrudable comprend 95% en poids de son poids d'un polymère de polypropylène A obtenu par polymérisation en présence d'un catalyseur de polymérisation à base de métallocène et 5% en poids de son poids d'un polymère de polypropylène B obtenu par polymérisation en présence d'un catalyseur de polymérisation Ziegler-Natta.

L'exemple 19 est un article de 40 g/m² comprenant quatre non-tissés spunbond mixed polymer de 10 g/m² (1,17 dtex / filament), la composition extrudable comprend 90% en poids de son poids d'un polymère de polypropylène A obtenu par polymérisation en présence d'un catalyseur de polymérisation à base de métallocène et 10% en poids de son poids d'un polymère de polypropylène B obtenu par polymérisation en présence d'un catalyseur de polymérisation Ziegler-Natta.

L'exemple 20 est un article de 64 g/m² comprenant quatre non-tissés spunbond mixed polymer de 16 g/m² (1,02 dtex / filament), la composition extrudable comprend 95% en poids de son poids d'un polymère de polypropylène A obtenu par polymérisation en présence d'un catalyseur de polymérisation à base de métallocène et 5% en poids de son poids d'un polymère de polypropylène B obtenu par polymérisation en présence d'un catalyseur de polymérisation Ziegler-Natta. Les dessins de la gravure sont en T.

L'exemple 21 est un article de 64 g/m² comprenant quatre non-tissés spunbond mixed polymer de 16 g/m² (1,03 dtex / filament), la composition extrudable comprend 90% en poids de son poids d'un polymère de polypropylène A obtenu par polymérisation en présence d'un catalyseur de polymérisation à base de métallocène et 10% en poids de son poids d'un polymère de polypropylène B obtenu par polymérisation en présence d'un catalyseur de polymérisation Ziegler-Natta. Les dessins de la gravure sont en pointes de diamant.

La différence entre les forces à rupture obtenues pour les soudures entre les exemples 16 et 17 démontrent très clairement que la sélection d'un polymère de polypropylène A obtenu par polymérisation en présence d'un catalyseur à base de métallocène permet très nettement d'améliorer la résistance des zones de solidarisation obtenues et ce quelle que soit la température de la soudure comparativement à l'emploi du polymère de polypropylène A obtenu par polymérisation en présence d'un catalyseur de polymérisation Ziegler-Natta.

Les résultats des forces à rupture obtenues pour les exemples 18 et 19 permettent de mettre en évidence l'amélioration de la résistance de la zone de solidarisation pour une composition extrudable comprenant environ 95% en poids de son poids d'un polymère de polypropylène A (métallocène) comparativement à une composition extrudable comprenant environ 90% en poids de son poids dudit polymère A.

Enfin, les exemples 20 et 21 démontrent clairement que la force à rupture obtenue pour la zone de solidarisation est encore améliorée par le choix de la température de solidarisation, en particulier entre 153°C et 155°C.

## Revendications

1. Non-tissé de filaments thermoplastiques filés-liés **caractérisé en ce qu'**il comprend au moins 95% en masse (g) de sa masse surfacique (g/m²) d'au moins deux polymère(s) de polypropylène A et B, les dits filaments ayant un titrage inférieur ou égal à 1,3 dtex, ledit non-tissé ayant une masse surfacique inférieure ou égale à 35 g/m², le taux de soudure étant au moins de 10% et au plus de 25% et **en ce qu'**il comprend au moins 88% en masse (g) de sa masse surfacique (g/m²) dudit polymère de polypropylène A obtenu par polymérisation en présence d'au moins un catalyseur de polymérisation à base de métallocène et au plus 12% en masse (g) de sa masse surfacique dudit polymère de polypropylène B.

2. Non-tissé selon la revendication 1, **caractérisé en ce qu'**il comprend au moins 93% en masse de sa masse surfacique dudit polymère de polypropylène A.

3. Non-tissé selon l'une ou l'autre des revendications 1 et 2, **caractérisé en ce que** les filaments ont un titrage compris entre 0,01 dtex et 1,2 dtex, de préférence entre 0,5 dtex et 1,1 dtex.

4. Non-tissé selon l'une quelconque des revendications 1 à 3, **caractérisé en ce qu'**il présente une masse surfacique d'au moins 5g/m² et d'au plus 30 g/m², de préférence d'au moins 10 g/m² et d'au plus 25 g/m².

5. Non-tissé selon l'une quelconque des revendications 1 à 4, **caractérisé en ce qu'**il a une perméabilité (l/m²/s) mesurée selon la norme ISO 9237 datant de 1995 inférieure à 6500 l/m²/s, de préférence inférieure à 6100 l/m²/s.

6. Article (1,6) comprenant au moins un premier non-tissé selon l'une quelconque des revendications 1 à 5 et au moins un second non-tissé comprenant des fibres et/ou des filaments à base d'un polymère de polypropylène, lesdits premier et second non-tissés sont solidarisés selon au moins une zone de solidarisation (4,5,7a,7b,7c,7d,11a,12a) dans laquelle ils sont thermo-liés.

7. Article (1,6) selon la revendication 6, **caractérisé en ce que** ladite zone de solidarisation (4,5,7a,7b,7c,7d,11a,12a) comprend au moins deux premiers non-tissés, de préférence au moins quatre premiers non-tissés.

8. Article d'hygiène, telle qu'une couche-culotte (1,6), adapté pour être porté autour du torse inférieur d'un porteur, ayant un axe longitudinal (L), et comprenant :
- une couche avant (3,8) perméable aux liquides,
- une couche arrière (2,7) imperméable aux liquides, et
- une couche absorbante disposée entre les couches avant (3,8) et arrière (2,7),
**caractérisé en ce que** la couche arrière (2,7) imperméable aux liquides comprend un film polymère imperméable aux liquides orienté vers ledit porteur et un premier non-tissé selon l'une quelconque des revendications 1 à 5.

9. Article d'hygiène selon la revendication 8, **caractérisé en ce que** le film polymère imperméable aux liquides et ledit premier non-tissé sont assemblés selon au moins une zone de solidarisation dans laquelle ledit film et ledit premier non-tissé sont thermo-liés.

10. Article d'hygiène (6) selon l'une ou l'autre des revendications 8 et 9, **caractérisé en ce qu'**il comprend un élément d'attache (9,10) solidarisé avec ledit premier non-tissé selon au moins une zone de solidarisation (9a,10a) dans laquelle ledit élément d'attache (9,10) et ledit premier non-tissé sont thermo-liés.

11. Article d'hygiène (1) selon l'une ou l'autre des revendications 8 et 9, **caractérisé en ce que** la couche arrière (2) comprend des portions avant (2a) et arrière (2b) solidarisées selon des zones de solidarisation latérales (4,5) dans lesquelles lesdites portions avant (2a) et arrière (2b) sont thermo-liées.

12. Article d'hygiène (6) selon l'une quelconque des revendications 8 à 10, **caractérisé en ce qu'**il comprend au moins une patte intermédiaire (11,12) d'attache disposée entre un élément d'attache (9,10) et la couche arrière (7), la patte intermédiaire d'attache (11,12) et la couche arrière (7) étant solidarisées selon une zone de solidarisation (11a,12a) dans laquelle elles sont thermo-liées.

13. Procédé de fabrication d'un non-tissé comprenant des filaments thermoplastiques filés-liés selon l'une des revendications 1 à 5, comprenant les étapes suivantes :
a. Extruder à travers les orifices d'une filière une composition à base de polypropylène comprenant au moins 95% en poids de son poids d'un mélange de deux polymères de polypropylène A et B, le polymère de polypropylène A étant présent à une teneur d'au moins 88 % en poids par rapport au poids de ladite composition et ayant un indice d'écoulement à l'état fondu d'au moins 10 g/10 min et au plus de 35 g/10 min, et le polymère de polypropylène B étant présent à une teneur d'au plus 12% en poids par rapport au poids de ladite composition et ayant un indice d'écoulement à l'état fondu d'au moins 600 g/10 min et au plus de 3 000 g/10 min , l'indice d'écoulement à l'état fondu étant déterminé d'après la norme ISO 1133, condition L, à 230°C et 2,16 Kg,
b. Etirer et napper les filaments extrudés sur un support,
c. Calandrer lesdits filaments pour la formation d'un premier non-tissé,
**caractérisé en ce que** le polypropylène A est obtenu par polymérisation en présence d'au moins un catalyseur de polymérisation à base de métallocène.

14. Procédé de fabrication selon la revendication 13, **caractérisé en ce que** la composition comprend au moins 93% en poids dudit polymère de polypropylène A, et au plus 7% en poids dudit polymère de polypropylène B, de préférence de l'ordre de 3% à 6% en poids dudit polymère de polypropylène B.

15. Procédé de fabrication selon l'une ou l'autre des revendications 13 et 14, pour la fabrication d'un article stratifié selon l'une ou l'autre des revendications 6 et 7 comprenant les étapes suivantes :
d. Superposer au moins un premier non-tissé obtenu à l'issue de l'étape c) et un second non-tissé,
e. Solidariser à l'aide d'un outil chauffé à une température comprise entre 150°C et 158°C, de préférence entre 153°C et 155°C, lesdits au moins deux non-tissés selon au moins une zone de solidarisation dans laquelle les dits non-tissés sont thermo-liés.

## Patentansprüche

1. Vliesstoff aus thermoplastischen Spunbonded-Filamenten, **dadurch gekennzeichnet, dass** er wenigstens 95 Masse-% (g) seiner flächenbezogenen Masse (g/m²) von wenigstens zwei Polypropylen-Polymer(en) A und B umfasst, wobei die Filamente eine Feinheit kleiner oder gleich 1,3 dtex haben, wobei der Vliesstoff eine flächenbezogene Masse kleiner oder gleich 35 g/m² hat, wobei der Schweißanteil wenigstens 10 % und höchstens 25 % beträgt, und dass er wenigstens 88 Masse-% (g) seiner flächenbezogenen Masse (g/m²) des Polypropylen-Polymers A, das durch Polymerisation in Gegenwart wenigstens eines Metallocen-basierten Polymerisationskatalysators erhalten wird, sowie höchstens 12 Masse-% (g) seiner flächenbezogenen Masse des Polypropylen-Polymers B umfasst.

2. Vliesstoff nach Anspruch 1, **dadurch gekennzeichnet, dass** er wenigstens 93 Masse-% seiner flächenbezogenen Masse des Polypropylen-Polymers A umfasst.

3. Vliesstoff nach dem einen oder anderen der Ansprüche 1 und 2, **dadurch gekennzeichnet, dass** die Filamente eine Feinheit im Bereich zwischen 0,01 dtex und 1,2 dtex, vorzugsweise zwischen 0,5 dtex und 1,1 dtex haben.

4. Vliesstoff nach einem der Ansprüche 1 bis 3, **dadurch gekennzeichnet, dass** er eine flächenbezogene Masse von wenigstens 5 g/m² und von höchstens 30 g/m², vorzugsweise von wenigstens 10 g/m² und von höchstens 25 g/m² aufweist.

5. Vliesstoff nach einem der Ansprüche 1 bis 4, **dadurch gekennzeichnet, dass** er eine Durchlässigkeit (l/m²/s), gemessen nach der Norm ISO 9237 von 1995, von weniger als 6500 l/m²/s, vorzugsweise weniger als 6100 l/m²/s aufweist.

6. Artikel (1, 6), umfassend wenigstens einen ersten Vliesstoff nach einem der Ansprüche 1 bis 5 und wenigstens einen zweiten Vliesstoff, der Fasern und/oder Filamente auf der Basis eines Polypropylen-Polymers umfasst, wobei der erste und der zweite Vliesstoff entlang wenigstens eines Bereichs zur festen Verbindung (4, 5, 7a, 7b, 7c, 7d, 11 a, 12a), in dem sie thermoverbunden sind, fest verbunden sind.

7. Artikel (1, 6) nach Anspruch 6, **dadurch gekennzeichnet, dass** der Bereich zur festen Verbindung (4, 5, 7a, 7b, 7c, 7d, 11a, 12a) wenigstens zwei erste Vliesstoffe, vorzugsweise wenigstens vier erste Vliesstoffe umfasst.

8. Hygieneartikel, wie eine Windelhose (1, 6), der dazu ausgelegt ist, um den unteren Rumpf eines Trägers herum getragen zu werden, eine Längsachse (L) hat und umfasst:
- eine flüssigkeitsdurchlässige vordere Schicht (3, 8),
- eine flüssigkeitsundurchlässige hintere Schicht (2, 7) und
- eine zwischen der vorderen (3, 8) und der hinteren (2, 7) Schicht angeordnete absorbierende Schicht,
**dadurch gekennzeichnet, dass** die flüssigkeitsundurchlässige hintere Schicht (2, 7) einen in Richtung des Trägers gerichteten flüssigkeitsundurchlässigen Polymerfilm und einen ersten Vliesstoff nach einem der Ansprüche 1 bis 5 umfasst.

9. Hygieneartikel nach Anspruch 8, **dadurch gekennzeichnet, dass** der flüssigkeitsundurchlässige Polymerfilm und der erste Vliesstoff entlang wenigstens eines Bereichs zur festen Verbindung, in dem der Film und der erste Vliesstoff thermoverbunden sind, zusammengefügt sind.

10. Hygieneartikel (6) nach dem einen oder anderen der Ansprüche 8 und 9, **dadurch gekennzeichnet, dass** er ein Befestigungselement (9, 10) umfasst, das mit dem ersten Vliesstoff entlang wenigstens eines Bereichs zur festen Verbindung (9a, 10a), in dem das Befestigungselement (9, 10) und der erste Vliesstoff thermoverbunden sind, fest verbunden ist.

11. Hygieneartikel (1) nach dem einen oder anderen der Ansprüche 8 und 9, **dadurch gekennzeichnet, dass** die hintere Schicht (2) einen vorderen (2a) und einen hinteren (2b) Abschnitt umfasst, die entlang von seitlichen Bereichen zur festen Verbindung (4, 5), in denen der vordere (2a) und der hintere (2b) Abschnitt thermoverbunden sind, fest verbunden sind.

12. Hygieneartikel (6) nach einem der Ansprüche 8 bis 10, **dadurch gekennzeichnet, dass** er wenigstens eine Befestigungszwischenlasche (11, 12), die zwischen einem Befestigungselement (9, 10) und der hinteren Schicht (7) angeordnet ist, umfasst, wobei die Befestigungszwischenlasche (11, 12) und die hintere Schicht (7) entlang eines Bereichs zur festen Verbindung (11a, 12a), in dem sie thermoverbunden sind, fest verbunden sind.

13. Verfahren zur Herstellung eines thermoplastische Spunbonded-Filamente umfassenden Vliesstoffs nach einem der Ansprüche 1 bis 5, umfassend die folgenden Schritte:
a. Extrudieren, durch die Öffnungen einer Spinndüse, einer Zusammensetzung auf Polypropylenbasis, die wenigstens 95 Gew.-% ihres Gewichts von einer Mischung aus zwei Polypropylen-Polymeren A und B umfasst, wobei das Polypropylen-Polymer A in einem Gehalt von wenigstens 88 Gew.-% bezogen auf das Gewicht der Zusammensetzung vorliegt und einen Schmelzflussindex von wenigstens 10 g/10 Min. und höchstens von 35 g/10 Min. aufweist, und wobei das Polypropylen-Polymer B in einem Gehalt von höchstens 12 Gew.-% bezogen auf das Gewicht der Zusammensetzung vorliegt und einen Schmelzflussindex von wenigstens 600 g/10 Min. und höchstens von 3.000 g/10 Min. aufweist, wobei der Schmelzflussindex nach der Norm ISO 1133, Bedingung L, bei 230 °C und 2,16 kg bestimmt wird,
b. Verstrecken und Ablegen der extrudierten Filamente auf einem Träger,
c. Kalandrieren der Filamente zur Bildung eines ersten Vliesstoffs,
**dadurch gekennzeichnet, dass** das Polypropylen A durch Polymerisation in Gegenwart wenigstens eines Metallocen-basierten Polymerisationskatalysators erhalten wird.

14. Herstellungsverfahren nach Anspruch 13, **dadurch gekennzeichnet, dass** die Zusammensetzung wenigstens 93 Gew.-% des Polypropylen-Polymers A und höchstens 7 Gew.-% des Polypropylen-Polymers B, vorzugsweise in der Größenordnung von 3 bis 6 Gew.-% des Polypropylen-Polymers B umfasst.

15. Herstellungsverfahren nach dem einen oder anderen der Ansprüche 13 und 14, für die Herstellung eines Schichtartikels nach dem einen oder anderen der Ansprüche 6 und 7, umfassend die folgenden Schritte:
d. Übereinanderlegen wenigstens eines am Ende des Schrittes c) erhaltenen ersten Vliesstoffs und eines zweiten Vliesstoffs,
e. festes Verbinden der wenigstens zwei Vliesstoffe mit Hilfe eines auf eine Temperatur zwischen 150 °C und 158 °C, vorzugsweise zwischen 153 °C und 155 °C erhitzten Werkzeugs, entlang wenigstens eines Bereichs zur festen Verbindung, in dem die Vliesstoffe thermoverbunden sind.

## Claims

1. A non-woven of spunbonded thermoplastic filaments **characterized in that** it comprises at least 95% by mass (g) of it surface mass (g/m²) of at least two polypropylene A and B polymers, said filaments having a titer of less than or equal to 1.3 dtex, said non-woven having a surface mass of less than or equal to 35 g/m², the welding rate being at least 10% and at most 25% and **in that** it comprises at least 88% by mass (g) of its surface mass (g/m²) of said polypropylene A polymer obtained by polymerization in the presence of at least one polymerization catalyst based on metallocene and at most 12% by mass (g) of its surface mass of said polypropylene B polymer.

2. The non-woven according to claim 1, **characterized in that** it comprises at least 93% by mass of its surface mass of said polypropylene A polymer.

3. The non-woven according to either one of claims 1 and 2, **characterized in that** the filaments have a titer comprised between 0.01 dtex and 1.2 dtex, preferably between 0.5 dtex and 1.1 dtex.

4. The non-woven according to any of claims 1 to 3, **characterized in that** it has a surface mass of at least 5 g/m² and of at most 30 g/m², preferably at least 10 g/m² and at most 25 g/m².

5. The non-woven according to any of claims 1 to 4, **characterized in that** it has a perviousness (l/m²/s) measured according to the ISO 9237 standard dating from 1995 of less than 6,500 l/m²/s, preferably less than 6,100 l/m²/s.

6. An article (1, 6) comprising at least one first non-woven according to any of claims 1 to 5, and at least one second non-woven comprising fibers and/or filaments based on a polypropylene polymer, said first and second non-wovens are fastened to each other along at least one fastening area (4,5,7a,7b,7c,7d,11a,12a) in which they are thermobonded.

7. The article (1,6) according to claim 6, **characterized in that** said fastening area (4,5,7a,7b,7c,7d,11a,12a) comprises at least two first non-wovens, preferably at least four first non-wovens.

8. A hygiene article, such as a diaper (1,6) adapted so as to be worn around the lower waist of a wearer, having a longitudinal axis (L) and comprising:
- a front layer (3,8) pervious to liquids,
- a rear layer (2,7) impervious to liquids, and
- an absorbing layer positioned between the front (3,8) and rear (2,7) layers,
**characterized in that** the rear layer (2,7) impervious to liquids comprises a polymeric film impervious to liquids oriented towards said wearer and a first non-woven according to any of claims 1 to 5.

9. The hygiene article according to claim 8, **characterized in that** the polymeric film impervious to liquids and said first non-woven are assembled along at least one fastening area in which said film and said first non-woven are thermobonded.

10. The hygiene article (6) according to either one of claims 8 and 9, **characterized in that** it comprises a tie element (9, 10) fastened with said first non-woven according to at least one fastening area (9a,10a) wherein said tie element (9, 10) and said first non-woven are thermobonded.

11. The hygiene article (1) according to either one of claims 8 and 9, **characterized in that** the rear layer (2) comprises front (2a) and rear (2b) portions fastened along lateral fastening areas (4, 5) wherein said front (2a) and rear (2b) portions are thermobonded.

12. The hygiene article (6) according to any of claims 8 to 10, **characterized in that** it comprises at least one intermediate tying tab (11, 12) positioned between a tie element (9, 10) and the rear layer (7), the intermediate tying tab (11, 12) and the rear layer (7) being fastened along a fastening area (11a, 12a) in which they are thermobonded.

13. A method for manufacturing a non-woven comprising spunbonded thermoplastic filaments according to one of claims 1 to 5, comprising the following steps:
a. Extruding through the orifices of a die a composition based on polypropylene comprising at least 95% by weight of its weight of a mixture of two polypropylene A and B polymers, the polypropylene A polymer being present at a content of at least 88% by weight based on the weight of said composition and having a melt flow index of at least 10 g/10 min and at most 35 g/10 min, and the polypropylene B polymer being present at a content of at most 12% by weight based on the weight of said composition and having a melt flow index of at least 600 g/10 min and at most 3,000 g/10 mins, the melt flow index being determined according to the ISO 1133 standard, condition L, at 230°C and with 2.16 kg,
b. Stretching and webbing the extruded filaments on a support,
c. Calendering said filaments for forming a first non-woven,
**characterized in that** the polypropylene A is obtained by polymerization in the presence of at least one polymerization catalyst based on a metallocene.

14. The manufacturing method according to claim 13, **characterized in that** the composition comprises at least 93% by weight of said polypropylene A polymer, and at most 7% by weight of said polypropylene B polymer, preferably of the order of 3% to 6% by weight of said polypropylene B polymer.

15. The manufacturing method according to any of claims 13 to 14, for manufacturing a laminated article according to either one of claims 6 and 7 comprising the following steps:
d. Superposing at least one first non-woven obtained at the end of step c) and a second non-woven,
e. Fastening by means of a tool heated to a temperature comprised between 150°C and 158°C, preferably between 153°C and 155°C, said at least two non-wovens along at least one fastening area in which said non-wovens are thermobonded.
